# EUROPÄISCHE PATENTSCHRIFT

(11) **EP 2 571 566 B1**
(45) Veröffentlichungstag und Bekanntmachung des Hinweises auf die Patenterteilung: **02.08.2017**
(21) Anmeldenummer: 11722005.3
(22) Anmeldetag: 16.05.2011
(51) Int. Cl.: A61M 39/22

(54) **VENTILANORDNUNG ZUR VERWENDUNG IN EINEM EXTRAKORPORALEN BLUTKREISLAUF, BLUTSCHLAUCHSATZ SOWIE BEHANDLUNGSVORRICHTUNG**
VALVE ARRANGEMENT FOR USE IN AN EXTRACORPOREAL BLOOD CIRCUIT, BLOOD TUBING SET AND A BLOOD TREATMENT DEVICE
ENSEMBLE DE VANNES DESTINÉ À ÊTRE UTILISÉ DANS UNE CIRCULATION DE SANG EXTRACORPORELLE, UN SET DE LIGNES À SANG ET UNE DISPOSITIF DE TRAITMENT DE SANG

(30) Priorität: 18.05.2010 DE 102010020838
(43) Veröffentlichungstag der Anmeldung: 27.03.2013
(73) Patentinhaber: Fresenius Medical Care Deutschland GmbH, 61352 Bad Homburg (DE)
(72) Erfinder: BACHMANN, Angelika, 61191 Rosbach v.d.H. (DE); HERRENBAUER, Michael, 61267 Neu-Anspach (DE); WEHMEYER, Wolfgang, 72076 Tübingen (DE)
(74) Vertreter: Bobbert & Partner Patentanwälte PartmbB
(86) Internationale Anmeldenummer: PCT/EP2011/002412
(87) Internationale Veröffentlichungsnummer: WO 2011/144315

(56) Entgegenhaltungen:
- WO-A1-2008/028579
- DE-A1-102008 059 379
- US-A- 5 957 879
- US-A1- 2003 018 290
- US-B1- 6 189 388

## Beschreibung

Die vorliegende Erfindung betrifft eine Ventilanordnung gemäß Anspruch 1. Sie betrifft ferner einen Blutschlauchsatz gemäß Anspruch 13 sowie eine Behandlungsvorrichtung gemäß Anspruch 14.

Aus der Praxis sind extrakorporale Blutkreisläufe zur Verwendung bei der extrakorporalen Behandlung von Körperfluiden wie Blut bekannt. Derartige Blutkreisläufe werden regelmäßig vor ihrer ersten Benutzung zur Verdrängung von darin enthaltener Luft mit einer Flüssigkeit befüllt. Sie werden regelmäßig nach Abschluss der Behandlung mit einem Fluid befüllt, um Blut, welches bei Abschluss der Behandlung im extrakorporalen Blutkreislauf vorliegt, in das Gefäßsystem des Patienten zurückzuführen.

Aus der US 6 189 388 B1 ist eine Vorrichtung zum Messen von Volumenströmen von Flüssigkeiten durch eine arteriovenöse Fistel bekannt.

Aus der US 5 957 879 A ist ein Verfahren zum vorübergehenden Stilllegen eines Patientenherzens und einer Blutentnahme zum Befüllen eines kardiovaskulären Bypasses bekannt.

Aus der US 2003/028579 A1 ist eine Durchfluss-Ventilvorrichtung mit mehreren Komponenten bekannt.

Aus der WO 2008/028579 A1 ist ein Blutbehandlungsgerät und ein Verfahren zum Entleeren eines Blutschlauchsatzes eines Blutbehandlungsgerätes bekannt.

Eine Aufgabe der vorliegenden Erfindung ist, eine neue Ventilanordnung vorzuschlagen, mittels welcher ein Kurzschließen von arterieller und venöser Leitung des extrakorporalen Blutkreislaufs, das Befüllen des Blutkreislaufs und/oder sein Entleeren möglich ist.

Diese Aufgabe wird durch eine Ventilanordnung mit den Merkmalen des Anspruchs 1 gelöst, durch einen Blutschlauchsatz gemäß Anspruch 13 sowie eine Blutkassette gemäß Anspruch 14.

Erfindungsgemäß wird damit eine Ventilanordnung vorgeschlagen, welche geeignet und/oder vorgesehen und/oder konfiguriert ist zu ihrer Verwendung in einem extrakorporalen Blutkreislauf, welcher wenigstens eine arterielle Blutleitung und wenigstens eine venöse Blutleitung aufweist. Die Ventilanordnung weist wenigstens ein erstes Ventil, welches in der arteriellen Blutleitung angeordnet ist, ein zweites Ventil, welches in der venösen Blutleitung angeordnet ist, ein viertes Ventil, welches in einer ersten arteriovenösen Verbindungsleitung zwischen arterieller Blutleitung des extrakorporalen Blutkreislaufs und venöser Blutleitung des extrakorporalen Blutkreislaufs angeordnet ist, sowie ein fünftes Ventil, welches in einer zweiten arteriovenösen Verbindungsleitung zwischen der arteriellen Blutleitung und der venösen Blutleitung angeordnet ist, auf.

Ergänzend zu den vorgenannten Ventilen weist die erfindungsgemäße Ventilanordnung ein drittes Ventil auf, welches zum Herstellen einer Fluidverbindung in einer Blutleitung zwischen arterieller Blutleitung und venöser Blutleitung des extrakorporalen Blutkreislaufs oder zum Herstellen einer Fluidverbindung direkt zwischen arterieller Blutleitung und venöser Blutleitung, vorzugsweise ohne zwischen geschaltetes Schlauchelement, angeordnet ist.

Luftablasseinrichtung des extrakorporalen Blutkreislaufs angeordnet ist.

Der erfindungsgemäße Blutschlauchsatz weist ebenso wie die erfindungsgemäße Blutkassette wenigstens eine erfindungsgemäße Ventilanordnung auf.

Die erfindungsgemäße Behandlungsvorrichtung ist zum extrakorporalen Behandeln medizinischer Fluide, insbesondere Blut, vorgesehen. Sie weist wenigstens eine Steuer- oder Regeleinrichtung sowie Aktoren auf, die dazu vorgesehen und konfiguriert sind, um wenigstens eine erfindungsgemäße Ventilanordnung zu steuern oder zu regeln.

Vorteilhafte Weiterbildungen der vorliegenden Erfindung sind jeweils Gegenstand der Unteransprüche.

Erfindungsgemäße Ausführungsformen können einige oder alle der folgenden Merkmale in beliebiger Kombination aufweisen.

In manchen erfindungsgemäßen Ausführungsformen ist die Blutbehandlungseinrichtung ein Blutfilter.

In bestimmten erfindungsgemäßen Ausführungsformen ist die Luftablasseinrichtung eine Tropfkammer oder Entlüftungsstelle oder jeweils Teil hiervon.

In manchen erfindungsgemäßen Ausführungsformen ist die arterielle Blutleitung des extrakorporalen Blutkreislaufs eine Leitung, welche während der Blutbehandlung sauerstoffreicheres Blut führt (gemessen an der venösen Blutleitung, welche folglich sauerstoffärmeres Blut führt).

Der erfindungsgemäße Blutschlauchsatz weist ebenso wie die erfindungsgemäße Blutkassette wenigstens eine erfindungsgemäße Ventilanordnung auf.

Die erfindungsgemäße Behandlungsvorrichtung ist zum extrakorporalen Behandeln medizinischer Fluide, insbesondere Blut, vorgesehen. Sie weist wenigstens eine Steuer- oder Regeleinrichtung sowie Aktoren auf, die dazu vorgesehen und konfiguriert sind, um wenigstens eine erfindungsgemäße Ventilanordnung zu steuern oder zu regeln.

Vorteilhafte Weiterbildungen der vorliegenden Erfindung sind jeweils Gegenstand der Unteransprüche.

Erfindungsgemäße Ausführungsformen können einige oder alle der folgenden Merkmale in beliebiger Kombination aufweisen.

In gewissen erfindungsgemäßen Ausführungsformen weist die erfindungsgemäße Ventilanordnung ein sechstes Ventil auf, welches zwischen einer Blutbehandlungseinrichtung und einer Luftablasseinrichtung des extrakorporalen Blutkreislaufs angeordnet ist.

In manchen erfindungsgemäßen Ausführungsformen ist die Blutbehandlungseinrichtung ein Blutfilter.

In bestimmten erfindungsgemäßen Ausführungsformen ist die Luftablasseinrichtung eine Tropfkammer oder Entlüftungsstelle oder jeweils Teil hiervon.

In manchen erfindungsgemäßen Ausführungsformen ist die arterielle Blutleitung des extrakorporalen Blutkreislaufs eine Leitung, welche während der Blutbehandlung sauerstoffreicheres Blut führt (gemessen an der venösen Blutleitung, welche folglich sauerstoffärmeres Blut führt).

In bestimmten erfindungsgemäßen Ausführungsformen ist die arterielle Blutleitung des extrakorporalen Blutkreislaufs eine Leitung, welche während der Blutbehandlung Blut vom Patienten wegführt, wohingegen die venöse Blutleitung Blut zum Patienten hinführt.

In manchen erfindungsgemäßen Ausführungsformen ist die arterielle Blutleitung des extrakorporalen Blutkreislaufs eine Leitung oder Patientenleitung, welche während der Blutbehandlung Blut zwischen einem Patientenkonnektor und dem Blutfilter führt. In bestimmten erfindungsgemäßen Ausführungsformen ist die venöse Blutleitung eine Leitung oder Patientenleitung, welche Blut zwischen einer Tropfkammer und einem Patientenkonnektor führt.

In manchen erfindungsgemäßen Ausführungsformen ist die erste und/oder die zweite arteriovenöse Blutleitung eine Fluidverbindung, beispielsweise als ein Schlauchabschnitt, eine Leitung oder dergleichen ausgestaltet, durch welche hindurch Blut von einer arteriellen Blutleitung zu einer venösen Blutleitung jeweils des extrakorporalen Blutkreislaufs fließen kann, gleich in welcher Richtung dies geschieht.

In bestimmten erfindungsgemäßen Ausführungsformen weist die Ventilanordnung zusätzlich wenigstens ein Entlüftungsventil zum Entlüften des Blutkreislaufs auf, insbesondere durch Herstellen einer Verbindung zwischen einem Inneren des extrakorporalen Blutkreislaufs und einem Äußeren hiervon, z.B. der Atmosphäre.

In manchen erfindungsgemäßen Ausführungsformen sind manche oder alle der oben genannten Ventile aktive Ventile.

Ein aktives Ventil im Sinne der vorliegenden Erfindung ist in bestimmten erfindungsgemäßen Ausführungsformen ein Ventil, welches, insbesondere ausschließlich, zum aktiven Betätigen vorgesehen ist, sei es manuell oder mittels geeigneter Einrichtungen. In diesen Ausführungsformen unterscheidet sich ein aktives Ventil somit von einem Ventil, welches sich selbsttätig öffnet oder schließt, wie z.B. ein Rückschlagventil dies tut.

Ein aktives Ventil im Sinne der vorliegenden Erfindung ist in bestimmten erfindungsgemäßen Ausführungsformen ein Ventil, welches, insbesondere ausschließlich, von einer Einrichtung, z.B. einem Controller, gesteuert oder betätigt wird.

Die Ventilanordnung weist in bestimmten erfindungsgemäßen Ausführungsformen eine Einrichtung zum Steuern oder Regeln der Ventilfunktion von allen oder manchen der oben genannten Ventile auf.

In manchen erfindungsgemäßen Ausführungsformen der Ventilanordnung ist diese ausgestaltet und vorgesehen zur Verwendung mit einer nicht-okkludierenden Pumpe zum Fördern des zu behandelnden Fluids, insbesondere von Blut.

In bestimmten erfindungsgemäßen Ausführungsformen der Ventilanordnung liegen wenigstens das erste, das zweite, das vierte und das fünfte Ventil in einem gemeinsamen Trägermaterial vor. Ein Trägermaterial im Sinne der vorliegenden Erfindung ist in bestimmten Ausführungsformen ein einziges Element, eine kleinste zerstörungsfrei vorliegende Einheit, eine Blutkassette, ein Adapter oder eine Einrichtung allgemein vorgesehen zum Verbinden arterieller und venöser Patientenleitungen oder -konnektoren oder dergleichen.

Die erfindungsgemäße Ventilanordnung umfasst in bestimmten Ausführungsformen wenigstens einen Rezirkulationsadapter oder ist wenigstens teilweise Teil eines solchen Rezirkulationsadapters. Der Rezirkulationsadapter weist in diesen Ausführungsformen wenigstens vier Ventile, insbesondere das erste, das zweite, das vierte und das fünfte Ventil, auf. In bestimmten Ausführungsformen weist der Rezirkulationsadapter ferner das dritte Ventil auf.

In manchen erfindungsgemäßen Ausführungsformen der Ventilanordnung ist wenigstens das dritte Ventil als Phantomventil ausgestaltet.

Ein "Phantomventil", wie hierin verwendet, kann ein Element mit einer mittels Aktor erreichbaren Aktor-Fläche (beispielsweise einer Aktor-Membran) sein, welches die Funktion eines Ventils übernehmen kann. Beispiele für geeignete Phantomventile können den Anmeldungen der vorliegenden Anmelderin DE 10 2009 018 664 A1, die am 23. April 2009 beim Deutschen Patent- und Markenamt hinterlegt wurde, DE 10 2009 024 468 A1, die am 10. Juni 2009 beim Deutschen Patent- und Markenamt hinterlegt wurde, oder der DE 10 2009 012 632 A1, die am 10. März 2009 beim Deutschen Patent- und Markenamt hinterlegt wurde, entnommen werden, auf deren diesbezügliche Offenbarung hiermit jeweils vollinhaltlich Bezug genommen wird.

In bestimmten erfindungsgemäßen Ausführungsformen weist die Ventilanordnung Sensoren auf zum Messen des arteriellen Drucks vor der Filtereinrichtung, z.B. einem Dialysator, ("pre filter pressure"), zum Messen des venösen Drucks stromabwärts der Luftablasseinrichtung, z.B. einer Luftabscheidekammer, ("pressure return"), zum Messen der optischen Dichte, zum Erfassen der in einem Blutleitungsinneren der arteriellen oder der venösen Blutleitung befindlichen Luftblasen ("Luftblasendetektor", air bubble detector, ABD), und dergleichen sowie beliebige Kombinationen hiervon. Die Sensoren können in oder an der arteriellen und/oder venösen Blutleitung vorliegen. Sie können in oder am arteriellen und/oder venösen Schenkel des extrakorporalen Blutkreislaufs vorliegen. Die Sensoren können als Multisensoren zum Messen/Erfassen mehrerer der vorgenannten Parameter ausgestaltet sein.

In manchen erfindungsgemäßen Ausführungsformen weist die Ventilanordnung eine Einrichtung zum Steuern oder Regeln einer Flussdifferenz auf, welche mittels, insbesondere gezieltem, Einstellen oder Betätigen einer Blutpumpe und/oder einer Dialysatpumpe und/oder geeigneter Einrichtungen wie Ventilen, Drosseln und dergleichen erzeugt wird.

In bestimmten erfindungsgemäßen Ausführungsformen ist die Ventilanordnung in einer Blutkassette ausgestaltet, oder Teil einer solchen, oder weist eine solche auf. Eine solche Blutkassette kann zum Beispiel als Gussteil oder Spritzgussteil ausgestaltet sein. Sie kann unabhängig hiervon als Einwegartikel ausgestaltet sein.

In manchen erfindungsgemäßen Ausführungsformen der Ventilanordnung sind ein, zwei oder mehr Ventile als Abschnitt eines drehbaren Elements bzw. eines Rotationselements schaltbar ausgestaltet. Dabei können die betreffenden drehbaren Elemente unabhängig von einander oder miteinander gekoppelt, also nur gemeinsam, betätigbar ausgestaltet sein.

Die erfindungsgemäße Behandlungsvorrichtung ist in bestimmten Ausführungsformen als extrakorporale Behandlungsvorrichtung, insbesondere als Dialysevorrichtung, insbesondere als Hämodialysevorrichtung, Hämofiltrationsvorrichtung, Hämodiafiltrationsvorrichtung oder als Vorrichtung für die Adsorption, Leberersatztherapie, Apherese, Transfusion, usw. ausgestaltet. Sie eignet sich besonders gut zum Einsatz bei Verfahren, bei welchen das Fluid mittels verschiedener Nadeln oder Zugänge dem Patienten entnommen und rückgeführt wird (letztere sind als double-needle-Verfahren bekannt).

Offenbart wird ein Verfahren zum Handhaben eines extrakorporalen Blutkreislaufs, bei welchem eine erfindungsgemäße Ventilanordnung verwendet wird. Das Handhaben des extrakorporalen Blutkreislaufs kann ein Kurzschließen einer arteriellen und einer venösen Blutleitung des extrakorporalen Blutkreislaufs, ein Befüllen des extrakorporalen Blutkreislaufs und/oder ein Freispülen von Abschnitten des extrakorporalen Blutkreislaufs mit oder ohne Flussstörung in einer der Blutleitungen des extrakorporalen Blutkreislaufs sowie beliebige Kombinationen hiervon umfassen oder hieraus jeweils bestehen.

Das Verfahren zum Handhaben dient in manchen Ausgestaltungen zum Kurzschließen einer arteriellen Blutleitung und einer venösen Blutleitung eines extrakorporalen Blutkreislaufs, mittels einer erfindungsgemäßen Ventilanordnung, unter Verbinden der
arteriellen Blutleitung mit der venösen Blutleitung durch Öffnen des dritten Ventils.

Das Verfahren zum Handhaben dient in manchen Ausgestaltungen zum Befüllen oder Primen eines extrakorporalen Blutkreislaufs oder von Teilen hiervon mittels einer erfindungsgemäßen Ventilanordnung unter Verwendung einer okkludierenden Blutpumpe. Dieses Verfahren kann die folgenden Schritte, einzeln oder in beliebiger Kombination, umfassen: Verbinden des extrakorporalen Blutkreislaufs mit einer okkludierenden Blutpumpe; Verbinden eines Dialysatkreislaufs mit einer Dialysatpumpe; Befüllen des Dialysatkreislaufs mit Fluid; Fördern eines Flusses mittels der Blutpumpe entgegen der bei einer extrakorporalen Blutbehandlung üblichen Flussrichtung; Fördern eines Flusses über die Membran eines Filters einer Blutbehandlungsvorrichtung mittels der Dialysatpumpe, welcher größer ist als der von der Blutpumpe geförderte oder erzeugte Fluss, bei geöffneten ersten und zweiten Ventilen; Öffnen eines Entlüftungsventils; Anhalten der Dialysatpumpe oder Einstellen eines Bilanziersystems auf neutrale Bilanz und Schließen des Entlüftungsventils; Zirkulieren des Fluids mit Hilfe der Blutpumpe bei weiterhin geöffneten ersten und zweiten Ventilen im extrakorporalen Blutkreislauf in üblicher Flussrichtung; Entfernen von Luft aus dem extrakorporalen Blutkreislauf mit Hilfe einer Luftabscheidekammer durch Einstellen einer positiven Bilanz, d.h. durch Erzeugen eines mittels der Dialysatpumpe erzeugten Flusses größer Null, im Bilanziersystem bei geöffnetem Entlüftungsventil.

Erfindungsgemäß wird in manchen Ausführungsformen der vorliegenden Erfindung unter einer "üblichen" Strömungs- oder Flussrichtung die bei einer extrakorporalen Blutbehandlung übliche Flussrichtung verstanden.

Dabei kann die bei einer extrakorporalen Blutbehandlung übliche Flussrichtung jene sein, in welcher das Blut extrakorporal die meiste Zeit strömt.

Die bei einer extrakorporalen Blutbehandlung übliche Flussrichtung kann jene sein, in welcher das Blut bei Double-Needle-Verfahren extrakorporal mittels der arteriellen Patienten- oder Blutleitung vom arteriellen Gefäßzugang des Patienten weg und mittels der venösen Patienten- oder Blutleitung zum venösen Gefäßzugang des Patienten hinströmt.

Dabei können vorzugsweise die Flüsse QD (Dialysat) und QB (Blut) derart festgelegt werden, dass ein gleichzeitiges Entlüften einer Blutleitung zwischen einem Filter einer Blutbehandlungsvorrichtung und der Luftabscheidekammer und der arteriellen Blutleitung und der venösen Blutleitung erreicht wird.

Ferner können hierbei die vorgenannten Schritte des Anhaltens der Dialysatpumpe oder Einstellens eines Bilanziersystems auf neutrale Bilanz und Schließens des Entlüftungsventils, des Zirkulierens und des Entfernens von Luft mehrfach durchgeführt werden.

Als Fluss QD ist in manchen Ausführungsformen der Fluss des Dialysats aus dem Dialysatkompartiment des Dialysators durch die Membran hindurch in das Blutkompartiment des Dialysators zu verstehen.

In manchen erfindungsgemäßen Ausführungsformen ist QD damit zu unterscheiden vom gesamten Fluss des Dialysats, der von der Dialysatpumpe in das Dialysatkompartiment des Dialysators hineingepumpt wird.

In bestimmten erfindungsgemäßen Ausführungsformen der vorliegenden Erfindung wird der Fluss des Dialysats vor und nach dem Dialysator bilanziert (z.B. über bekannte Bilanzkammern, nicht gezeigt in den Figuren).

Zum Bilanzieren können geeignete Einrichtungen und/oder Verfahren bzw. Vorgehensweisen verwendet werden.

Das Bilanzieren während einer extrakorporalen Blutbehandlung kann insbesondere in Ausführungsformen, in denen dem Patienten während beispielsweise einer Dialyse eine definierte Menge an überschüssigem Wasser entzogen werden soll, von Interesse oder von Bedeutung sein.

Unter einer "neutralen Bilanz" wird in manchen erfindungsgemäßen Ausführungsformen verstanden, dass die Drücke und Flüsse vor und nach dem Dialysatkompartiment und im Blutkompartiment so eingestellt sind, dass kein Fluss QD des Dialysats durch die Membran hindurchgedrückt wird. Dies kann beispielsweise durch das vollständige Anhalten der Dialysatpumpe erfolgen. Ein Anhalten oder vollständiges Anhalten ist hierzu aber nicht zwingend erforderlich.

Bei einer neutralen Bilanz ist der Fluidfluss zum Patienten daher in bestimmten Ausführungsformen genauso groß wie der Fluidfluss vom Patienten weg. Das Patientengewicht kann daher - im Wesentlichen oder vollständig - konstant bleiben.

Im Gegensatz dazu gilt bei einer "positiven Bilanz" in manchen Ausführungsformen der Erfindung, dass ein Fluss QD des Dialysats größer Null ist, d.h. QD>0. Bei QD>0 tritt Dialysat durch die Membran hindurch in das Blutkompartiment über. Anders ausgedrückt, werden im Dialysatsystem die Drücke und Flüsse vor und nach dem Dialysatkompartiment und im Blutkompartiment so eingestellt, dass ein Fluss QD des Dialysats vom Dialysatkompartiment durch die Membran in das Blutkompartiment hinein erzwungen wird.

Bei einer positiven Bilanz ist der Fluidfluss zum Patienten damit in bestimmten Ausführungsformen höher als der Fluidfluss vom Patienten weg. Der Patient kann damit bei positiver Bilanz Flüssigkeit zugeführt bekommen.

Die gewünschte "Bilanz" kann z.B. nach einem vorgegebenen Programm von der Steuerung der Blutbehandlungsmaschine eingestellt werden.

Unabhängig vom Einstellen einer positiven oder neutralen Bilanz können in manchen erfindungsgemäßen Ausführungsformen für die einzelnen Spülvorgänge qualitative Verhältnisse zwischen QD und QB vorgegeben sein oder werden. Durch ein solches Verhältnis kann unter bestimmten Umständen auch die "Bilanz" beeinflusst werden, muss aber nicht. Eine Einstellung des Verhältnisses zwischen QD und QB kann in bestimmten erfindungsgemäßen Ausführungsformen der Einstellung bestimmter Flüsse in der arteriellen und/oder der venösen Leitung und der erfindungsgemäßen Ventilanordnung (auch als "Spüladapter" bezeichnet) dienen.

Das Verfahren kann zum Handhaben ein Befüllen des extrakorporalen Blutkreislaufs oder von Teilen unter Einsatz einer nicht-okkludierenden Blutpumpe sein oder umfassen. Dieses Verfahren umfasst wenigstens einen der Schritte: Kurzschließen von Patientenkonnektoren; Einbringen von Fluid aus einem Dialysatkreislauf in den extrakorporalen Blutkreislauf durch Einstellen einer positiven Bilanz; Anhalten der Blutpumpe; Schließen eines sechsten Ventils, Öffnen des ersten und zweiten Ventils sowie des Entlüftungsventils; Schließen des dritten, vierten und fünften Ventils soweit vorhanden; Pressen von Fluid aus dem Dialysatkompartiment über die Membran des Filters auf die Blutseite des Filters; Schließen des ersten und des zweiten Ventils; Öffnen des sechsten Ventils nach Erkennen eines Fluidpegels in der Luftabscheidekammer; Anhalten der Dialysatpumpe und/oder Einstellen des Bilanziersystems auf neutrale Bilanz; Schließen des Entlüftungsventils; Zirkulieren des Fluids mit Hilfe der Blutpumpe bei geöffnetem ersten und geöffnetem zweiten Ventil sowie geöffnetem sechstem Ventil im extrakorporalen Blutkreislauf; Einstellen einer positiven Bilanz im Bilanziersystem (QD>0); Öffnen des Entlüftungsventils; und Anhalten der Blutpumpe.

Einige der Schritte dieses Verfahrens können mehrfach durchgeführt werden.

Das Verfahren kann zum Handhaben eines extrakorporalen Blutkreislaufs mittels der erfindungsgemäßen Ventilanordnung ein Freispülen eines extrakorporalen Blutkreislaufs oder von Teilen hiervon mittels einer erfindungsgemäßen Ventilanordnung sein oder umfassen, ohne Vorliegen einer Flussstörung in einer der Blutleitungen. Das Verfahren umfasst dann wenigstens einen der Schritte: Fördern eines Flusses mittels der Blutpumpe entgegen der üblichen Flussrichtung bei mit dem Gefäßzugang des Patienten verbundenen Blutleitungen; Fördern von Fluid mittels der Dialysatpumpe aus dem Dialysatkreislauf mit einem Fluss größer dem mittels der Blutpumpe erzeugten Fluss über die Membran eines Filters einer Blutbehandlungsvorrichtung in den extrakorporalen Blutkreislauf; Öffnen des ersten und des zweiten Ventils, Öffnen des sechsten Ventils bei geschlossenem dritten, vierten und fünften Ventil und geschlossenem Entlüftungsventil, sofern jeweils vorhanden; Feststellen mittels Sensoren, wann oder dass das Fluid das Blut verdrängt oder ausreichend verdrängt hat; Steuern oder Regeln des Blutrückgabeprozesses über die Höhe der Flussdifferenz zwischen Fluss, welcher mittels der Dialysatpumpe erzeugt oder gefördert wird, und Fluss, welcher mittels der Blutpumpe gefördert wird, oder durch entsprechendes Schalten des ersten und des zweiten Ventils; Anhalten der nicht-okkludierenden Blutpumpe; Schalten des ersten und des zweiten Ventils zum Veranlassen der Blutrückgabe über eine ausgewählte der Blutleitungen.

Das Verfahren kann zum Handhaben eines extrakorporalen Blutkreislaufs ein Freispülen des extrakorporalen Blutkreislaufs oder von Teilen hiervon sein oder umfassen, bei Vorliegen einer Flussstörung in einer der Blutleitungen. Das Verfahren umfasst dann wenigstens einen der Schritte: Fördern mittels der Blutpumpe mit einem Fluss entgegen der üblichen Flussrichtung, wobei die arterielle Blutleitung und/oder die venöse Blutleitung jeweils mit dem entsprechenden Gefäßzugang des Patienten verbunden sind; Fördern von Fluid aus dem Dialysatkreislauf über die Membran eines Filters einer Blutbehandlungsvorrichtung in den extrakorporalen Blutkreislauf mittels der Dialysatpumpe mit einem Fluss, welcher größer ist als der mittels der Blutpumpe erzeugte Fluss; Leiten des Gesamtflusses auf eine der beiden Blutleitungen durch entsprechendes Schalten des ersten, zweiten, dritten, vierten und/oder fünften Ventils; Schalten des fünften Ventils und des sechsten Ventils oder geeignetes Auswählen der Flussdifferenz zwischen dem mittels der Dialysatpumpe und dem mittels der Blutpumpe geförderten Fluss, zum Durchströmen der arteriellen Blutleitung oder der venösen Blutleitung.

Dabei kann beim Verfahren ferner ein Fluss QD im extrakorporalen Blutkreislauf erzeugt werden, mittels der Dialysatpumpe bei nicht fördernder oder stehender oder nicht überspülter Blutpumpe.

Ferner wird in bestimmten Ausführungsformen des Verfahrens mittels der Blutpumpe und mittels der Dialysatpumpe mit gleichen Flussraten der Pumpen gefördert, wobei die Blutpumpe vorzugsweise rückwärts dreht bzw. rückwärts fördert.

Erfindungsgemäß kann unter dem Begriff "rückwärts", wenn im Zusammenhang mit der Förderrichtung der Blutpumpe und/oder der Dialysatpumpe die Rede ist, eine Richtung, welche entgegengesetzt zur Förderrichtung der betroffenen Pumpe steht, in welche mittels der betroffenen Pumpe bei laufender Blutbehandlung überwiegend gefördert wird, oder eine Richtung, welche entgegengesetzt der bei laufender Blutbehandlung üblichen Förderrichtung ist, verstanden werden.

Das Verfahren umfasst in bestimmten Ausführungsformen ferner ein Einstellen einer positiven Bilanz, bei welcher der mittels der Dialysatpumpe geförderte Fluss größer als der mittels der Blutpumpe geförderte Fluss ist, bei Verwenden einer nicht-okkludierenden Blutpumpe und/oder ein Schalten der Flusswege durch die ersten bis
fünften Ventile sowie des sechsten Ventils zum Freispülen der nicht blockierten Blutleitungen.

Alle mit der erfindungsgemäßen Ventilanordnung erzielbaren Vorteile lassen sich ungeschmälert auch mit den Verfahren erzielen und umgekehrt.

Offenbart wird ein digitales Speichermedium, in Verbindung mit entsprechender, dazu geeigneter und/oder dazu vorgesehener Hardware, ein Computer-Programm-Produkt und ein Computer-Programm.

Das digitale Speichermedium, welches insbesondere eine Diskette, eine CD oder eine DVD, ein USB-Stick, eine Flashcard, eine SD-Karte usw. ist, weist bevorzugt elektrisch auslesbare Steuersignale auf, die so mit einem programmierbaren Computersystem zusammenwirken können, dass die maschinellen Schritte des Verfahrens veranlasst werden.

Dabei können alle, einige oder manche der maschinell durchgeführten Schritte des Verfahrens veranlasst werden. Letzteres gilt auch für das Computer-Programm-Produkt und das Computer-Programm.

Das Computer-Programm-Produkt weist vorzugsweise einen auf einem maschinenlesbaren Träger gespeicherten Programmcode auf zur Veranlassung der maschinell durchführbaren Schritte des Verfahrens, wenn das Programm-Produkt auf einem Rechner abläuft.

Der Begriff "maschinenlesbarer Träger", wie er hierin verwendet wird, bezeichnet einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen sein.

Das Computer-Programm weist einen Programmcode zur Veranlassung der maschinell durchführbaren Schritte des Verfahrens auf, wenn das Programm auf einem Rechner oder Computer abläuft.

Bestimmte erfindungsgemäße Ausführungsformen weisen einen oder mehrere der im Folgenden genannten Vorteile auf.

Die vorliegende Erfindung stellt eine Ventilanordnung bereit, mittels der ein Entlüften und/oder Freispülen eines extrakorporalen Blutkreislaufs vorteilhaft in einfacher und technisch wenig aufwendiger Weise möglich ist. Die erfindungsgemäße Ventilanordnung kann vorteilhaft auch bei einer nicht-okkludierenden Blutpumpe angewendet werden, wie es bislang für derartige Pumpen im Bereich der extrakorporalen Blutbehandlung nicht möglich war. Damit können einige der oben genannten Verfahren vorteilhaft nun auch bei Verwendung nicht-okkludierender Blutpumpen zum Einsatz kommen.

Mittels des dritten Ventils kann die Luftabscheidung aus dem extrakorporalen Blutkreislauf vorteilhaft durchgeführt oder begünstigt werden. Es kann während der Behandlung zum Eintritt von Luft in den extrakorporalen Blutkreislauf kommen. Diese Luft wird üblicherweise vom Schutzsystem (Air bubble detector) erkannt und muss aus dem extrakorporalen Blutkreislauf entfernt werden. Bei herkömmlichen Schlauchsystemen geschieht dies manuell.

Durch automatisiertes Entlüften, wie mittels der vorliegenden Erfindung möglich, kann der hierzu erforderliche Arbeitsaufwand vorteilhaft minimiert werden. In manchen erfindungsgemäßen Ausführungsformen kann die Systemsicherheit erhöht werden. Dies kann insbesondere dann der Fall sein, wenn zum Entlüften das Blut über den Ventilblock bzw. Rezirkulationsadapter vom Patientenkreislauf entkoppelt wird, vorzugsweise unter entsprechender Schaltung des dritten Ventils. Hierbei kann das extrakorporal vorliegende Blut innerhalb des extrakorporalen Blutkreislaufs so umgepumpt werden, dass die Luft direkt in die Luftabscheidekammer geleitet wird und dabei nicht etwa zum Patienten gelangt und auch nicht durch den Kapillarfilter, z.B. einer Dialysevorrichtung, geleitet werden muss. Dies kann besonders vorteilhaft sein, da sich Luft im Kapillarfilter leicht "festsetzt" oder zu Mikroblasen zerkleinert wird. Daher bietet das dritte Ventil vorteilhaft die Möglichkeit, vorhandene Luftblasen mittels interner Zirkulation oder Rezirkulation in oder entgegen der üblichen Flussrichtung einer extrakorporalen Blutbehandlung in die Luftabscheidekammer (z.B. die venöse Tropfkammer) zu leiten.

Das dritte Ventil kann vorteilhaft die Möglichkeit bieten, das Blut innerhalb der Kassette zu rezirkulieren, während der Patient entkoppelt ist. Dabei kann der Patient allerdings bei Wunsch sogar konnektiert bleiben, was vorteilhaft Zeit, Arbeit und Mühe ersparen kann.

Eine Rezirkulation innerhalb des extrakorporalen Blutkreislaufs kann den Vorteil bieten, dass extrakorporal vorliegendes Blut im Falle z.B. eines Zugangsproblems (z.B. bei hohem Rücklaufdruck), weiterhin über den Filter gepumpt werden kann. Auf diese Weise kann in bestimmten Ausführungsformen vorteilhaft vermieden werden, dass ein derartiges Zugangsproblem zu einem Stillstand der Strömung durch den extrakorporalen Blutkreislaufs führt. Dadurch kann es vorteilhaft möglich sein, das Risiko einer Koagulation des Blutes, beispielsweise im Filter, deutlich zu reduzieren.

Die vorliegende Erfindung kann ferner in manchen Ausführungsformen hiervon vorteilhaft dazu beitragen, dass in Alarmsituationen, in denen üblicherweise ein Blutpumpenstopp notwendig wäre, weiterhin eine Zirkulation über den Filter möglich bleibt. Auf diese Weise kann für den Patienten vorteilhaft sichergestellt werden, dass er keinen Blutverlust durch Verwerfen des koagulierten Systems erleidet, eine höhere effektive Behandlungszeit erzielt wird und ein ggf. erforderlicher Systemwechsel entfällt.

Wird das dritte Ventil zur Rezirkulation bzw. internen Zirkulation geöffnet, so werden in manchen erfindungsgemäßen Ausführungsformen - falls jeweils vorhanden - das sechste Ventil und, im Falle der Luftabscheidung, das Entlüftungsventil geöffnet sein. Das erste, zweite, vierte und fünfte Ventil sollte jeweils geschlossen sein.

Das sechste Ventil kann vorteilhaft verwendet werden, um sowohl die arterielle Blutleitung als auch die venöse Blutleitung bei Verwendung einer nicht-okkludierenden Blutpumpe füllen bzw. primen zu können. Dazu wird mittels der Dialysatpumpe zunächst retrograd, also gegen die übliche Flussrichtung, bei geschlossenem sechstem Ventil die arterielle Blutleitung, und nach Öffnen des sechsten Ventils mittels der Blutpumpe anterograd, also in üblicher Flussrichtung, die venöse Blutleitung befüllt.

Das sechste Ventil kann bei okkludierender Blutpumpe vorteilhaft dazu eingesetzt werden, die arterielle Blutleitung stromaufwärts zu spülen.

Das sechste Ventil kommt neben dem vorstehend beschriebenen Einsatz auch beim Freispülen (Blutrückgabe) des extrakorporalen Blutkreislaufs in analoger Weise zum Einsatz.

Die Positionierung des sechsten Ventils zwischen dem Filter und der Tropfkammer kann vorteilhaft dazu beitragen, die Luftabscheidung, das Befüllen und das Entleeren in beabsichtigter Weise durchzuführen.

Mittels des ersten und vierten sowie des zweiten und fünften Ventils kann vorteilhaft bei Bedarf die Flussrichtung in der arteriellen sowie der venösen Blutleitung umgekehrt werden (Flussumkehr). Dabei kann die Blutflussrichtung im Filter beibehalten werden. Auf diese Weise kann das Gegenstromprinzip vorteilhaft beibehalten werden. Die Flussumkehr kann insbesondere dann vorteilhaft sein, wenn Flussprobleme, z.B. durch Ansaugen des Katheters, entstehen. Die Flussumkehr kann in bestimmten erfindungsgemäßen Ausführungsformen durch das Dialysegerät automatisch oder selbsttätig (mittels entsprechender Einrichtungen) eingeleitet werden. Dies mag beispielsweise dann erfolgen, wenn ein nicht ausreichender Fluss detektiert wird.

Daneben bietet die erfindungsgemäße Ventilanordnung vorteilhaft die Möglichkeit, eine Blutrückgabe bei einseitigem Verschluss einer Blutleitung und/oder eines Katheters oder einer Nadel zu veranlassen.

Auch das Verwenden von aktiven Ventilen, wie in bestimmten Ausführungsformen der vorliegenden Erfindung vorgesehen, kann vorteilhaft ermöglichen das Gegenstromprinzip aufrecht zu erhalten.

Daneben können aktive Ventile vorteilhaft dazu beitragen, ein einseitiges Freispülen und/oder eine einseitige Blutrückgabe über die arterielle ("access") Blutleitung oder die venöse ("return") Blutleitung des Schlauchsystems zu ermöglichen. Auf diese Weise kann es vorteilhaft möglich sein, eine automatisierte Diagnose der Zugangsprobleme (Clotting/Ansaugen des Katheters) vorzunehmen, da an den beiden Blutleitungen (arterieller wie venöser Blutleitung) jeweils separat bzw. unabhängig voneinander vorteilhaft ein Fluss in eine oder gar beide Richtungen (mit und entgegen der üblichen Flussrichtung) erzeugt werden kann.

Im Folgenden wird die vorliegende Erfindung beispielhaft unter Bezugnahme auf die beigefügte Zeichnung beschrieben. In der Zeichnung bezeichnen identische Bezugszeichen gleiche oder identische Elemente. Es gilt:
- Fig. 1: zeigt eine erste erfindungsgemäße Ventilanordnung während eines Füllprozesses bei okkludierender Blutpumpe;
- Fig. 2: zeigt eine zweite erfindungsgemäße Ventilanordnung während eines Füllprozesses bei nicht-okkludierender Blutpumpe;
- Fig. 3: zeigt die zweite erfindungsgemäße Ventilanordnung während eines Füllprozesses zum Freispülen von Blutleitungen;
- Fig. 4: zeigt schematisch eine erfindungsgemäße Ventilanordnung in einer ersten Ausführungsform;
- Fig. 5: zeigt schematisch eine erfindungsgemäße Ventilanordnung in einer zweiten Ausführungsform; und
- Fig. 6: zeigt schematisch eine erfindungsgemäße Ventilanordnung in einer dritten Ausführungsform.

Die vorliegende Erfindung kann vorteilhaft bei einer Vielzahl extrakorporaler Blutbehandlungsverfahren, wie beispielsweise einer Nierenersatztherapie, einer Leberersatztherapie, einer Apherese, einer Transfusion und dergleichen eingesetzt werden, insbesondere bei denen der Zugang zum Blutsystem des Patienten mittels zweier Konnektionseinrichtungen, wie Nadeln oder Kanülen, hergestellt ist. Beispiele für solche Verfahren schließen Hämodialyse, Hämofiltration, Hämodiafiltration oder Adsorption ein.

Die vorliegende Erfindung wird im Folgenden beispielhaft anhand eines Dialyseverfahrens beschrieben, ohne die Erfindung in irgendeiner Weise auf ein solches Handhabungsverfahren oder die folgenden Ausführungsformen beschränken zu wollen.

Zur Durchführung des Dialyseverfahrens wird ein extrakorporaler Blutkreislauf eingesetzt. Der extrakorporale Blutkreislauf kann als Schlauchsystem ausgestaltet sein. Der extrakorporale Blutkreislauf wird von Fluiden durchströmt. Beispiele für den extrakorporalen Blutkreislauf durchströmende Fluide schließen Flüssigkeiten, wie Blut, Dialysat, Substituatflüssigkeit, Medikamentenlösungen und dergleichen, Gase, wie Luft, sowie Kombinationen oder Mischungen derselben ein.

In den im Folgenden erläuterten Figuren sind offene bzw. geöffnete Ventile durch ein Quadrat am Ventilsymbol dargestellt; geschlossene Ventile weisen kein solches Quadrat auf.

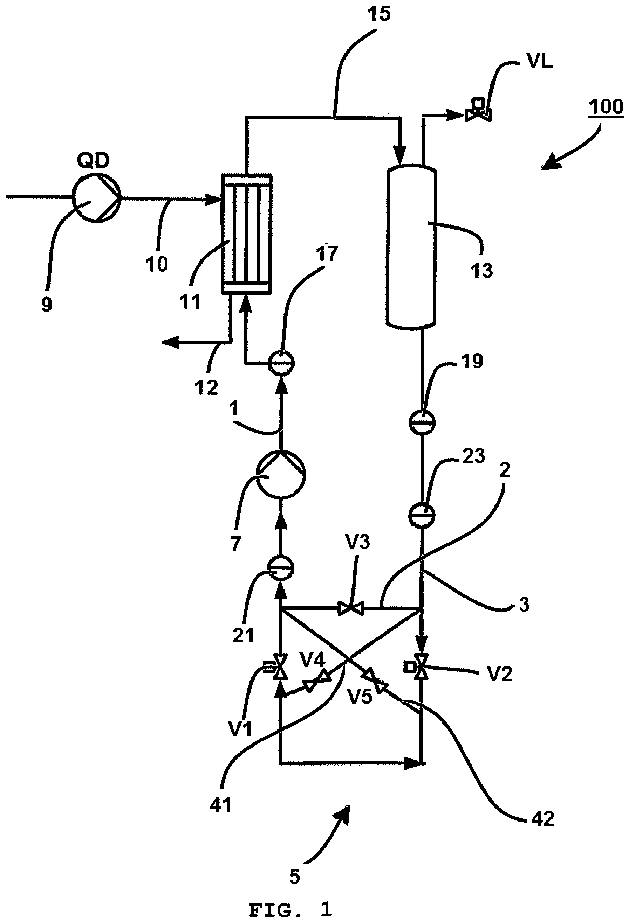

**Fig. 1** zeigt eine erste erfindungsgemäße Ventilanordnung während eines Füllprozesses bei okkludierender Blutpumpe in einem ersten Füllabschnitt.

Bei dem in Fig. 1 gezeigten Verfahren kann es sich um ein initiales Befüllen eines extrakorporalen Blutkreislaufs 100 handeln.

Der extrakorporale Blutkreislauf 100, der in Fig. 1 schematisch dargestellt ist, weist eine arterielle Patientenleitung bzw. arterielle Blutleitung 1 und eine venöse Patientenleitung bzw. venöse Blutleitung 3 auf.

Patientenkonnektoren, welche einen Zugang zum Gefäßsystem eines Patienten zu dessen extrakorporaler Blutbehandlung erlauben, z.B. ein arterieller und ein venöser Patientenzugang (in Fig. 1 nicht gezeigt) sind kurzgeschlossen. Wie in Fig. 1 gezeigt, sind die Patientenkonnektoren über eine erfindungsgemäße Ventilanordnung, in Fig. 1 z.B. einen Rezirkulationsadapter 5, kurzgeschlossen. Der Rezirkulationsadapter 5 weist fünf Ventile auf, wie in Fig. 1 gezeigt, nämlich das erste Ventil V1, das zweite Ventil V2, das dritte Ventil V3, das vierte Ventil V4 und das fünfte Ventil V5.

Das erste Ventil V1 ist in der arteriellen Blutleitung 1 angeordnet, das zweite Ventil V2 in der venösen Blutleitung 3, das dritte Ventil V3 ist in einer Blutleitung 2 zwischen der arteriellen Blutleitung 1 und der venösen Blutleitung 3 angeordnet, das vierte Ventil V4 ist in einer ersten arteriovenösen Blutleitung 41 angeordnet und das fünfte Ventil V5 in einer zweiten arteriovenösen Blutleitung 42.

Die vorgenannten Ventile werden im Folgenden kurz als Ventile V1 bis V5 bezeichnet. Ebenso wird das sechste Ventil V6 kurz als Ventil V6 bezeichnet.

In bzw. an der arteriellen Blutleitung 3 des extrakorporalen Blutkreislaufs 100 ist eine Blutpumpe 7 angeordnet. Die Blutpumpe 7 ist in der anhand von Fig. 1 erläuterten Ausführungsform der vorliegenden Erfindung als okkludierende Blutpumpe ausgestaltet.

Ein Dialysatkreislauf (in Fig. 1 durch eine Dialysatzuleitung 10 für frisches Dialysat und eine Dialysatableitung 12 für gebrauchtes Dialysat angedeutet), weist eine Dialysatpumpe 9 auf. Zur Durchführung des hier beschriebenen Verfahrens kann der Dialysatkreislauf vollständig mit Fluid, z.B. Dialysat, gefüllt sein. Er kann teilweise oder in Abschnitten mit Fluid gefüllt sein.

Die Blutpumpe 7 fördert einen Fluss bzw. Fluidstrom QB, welcher ein Volumen pro Zeit angibt.

In der vorliegenden Ausführungsform fördert die Blutpumpe 7 entgegen der bei einer extrakorporalen Blutbehandlung üblichen Flussrichtung (d.h. entgegen der Pfeilrichtung in Fig. 1). Gleichzeitig fördert die Dialysatpumpe 9 einen Fluss bzw. Fluidstrom QD, welcher ein Volumen pro Zeit angibt, mit QD>QB über die Membran eines Filters hinweg bzw. durch die Membran hindurch, wie die Membran eines Dialysators 11.

Als Dialysator 11 kann ein HD 1000 S-Dialysator der Fa. Fresenius Medical Care AG & Co. KG, Deutschland, eingesetzt werden. Die Art und/oder Ausgestaltung des Dialysators ist jedoch nicht besonders beschränkt.

QD ist der Fluss auf der Dialysatseite des Dialysators 11 über die Membran in das Blutkompartiment bzw. zur Blutseite des extrakorporalen Blutkreislaufs 100. Dazu muss ein Bilanziersystem (in Fig. 1 nicht dargestellt) entsprechend eine "positive" Bilanz mit einem Fluss QD (QD>QB) erzeugen.

Wie vorstehend angegeben, kann der Fluidfluss zum Patienten bei einer positiven Bilanz in manchen erfindungsgemäßen Ausführungsformen höher als der Fluidfluss vom Patienten weg sein. Der Patient kann bei positiver Bilanz Flüssigkeit zugeführt bekommen.

Die Ventile V1 und V2 sind geöffnet, die Ventile V3, V4 und V5 sind geschlossen. Die Ventile V3, V4 und V5 sind für die Funktion des in Fig. 1 veranschaulichten Füllprozesses nicht erforderlich bzw. müssen nicht vorgesehen sein.

Ein Entlüftungsventil VL ist geöffnet, welches, wie in Fig. 1 gezeigt, in einer Ableitung einer Luftablasseinrichtung, wie einer venösen Tropfkammer oder einer Luftabscheidekammer 13, der venösen Blutleitung 3 des extrakorporalen Blutkreislaufs 100 angeordnet ist. Andere Anordnungen des Entlüftungsventils VL sind möglich und von der vorliegenden Erfindung umfasst. Das Entlüftungsventil VL kann zu einem Äußeren des extrakorporalen Blutkreislaufs 100, insbesondere z.B. in die Umgebung oder zur Atmosphäre, hin entlüften.

Das über die Membran des Dialysators 11 in das Blutkompartiment des extrakorporalen Blutkreislaufs 100 eingebrachte Dialysat verdrängt zunächst im Dialysator 11 und im weiteren Verlauf sowohl stromaufwärts als auch stromabwärts Luft aus dem Blutkompartiment des extrakorporalen Blutkreislaufs 100. Diese Luft wird dabei in der Luftabscheidekammer 13 (z.B. venöse Tropfkammer oder spezifische Luftabscheidekammer) aufgefangen und abgeschieden.

Durch geeignetes Festlegen der Flüsse QD und QB bzw. durch Einstellen einer Flussdifferenz (QD-QB) kann ein gleichzeitiges Entlüften einer Blutleitung 15 zwischen dem Dialysator 11 und der Luftabscheidekammer 13 und der arteriellen und der venösen Blutleitung 1 und 3 erreicht werden.

Sobald die Blutleitungen 1, 2 und 3 und die Luftabscheidekammer 13 mit Fluid gefüllt sind, ist der erste Füllabschnitt beendet.

In einem zweiten Füllabschnitt des Verfahrens (in Fig. 1 nicht dargestellt) wird zunächst die Dialysatpumpe 9 angehalten bzw. das Bilanziersystem auf neutrale Bilanz eingestellt und das Entlüftungsventil VL geschlossen.

Wie vorstehend angegeben, kann der Fluidfluss zum Patienten bei einer neutralen Bilanz genauso groß wie der Fluidfluss vom Patienten weg sein. Das Patientengewicht kann daher konstant bleiben.

Das Fluid (z.B. Dialysat) wird mit Hilfe der Blutpumpe 7 bei weiterhin geöffneten Ventilen V1 und V2 im extrakorporalen Blutkreislauf 100 zirkuliert. Dabei fördert die Blutpumpe 7 in üblicher Flussrichtung (in der in Fig. 1 dargestellten Pfeilrichtung).

Dadurch wird im extrakorporalen Blutkreislauf 100 vorhandene Restluft in die Luftabscheidekammer 13 geleitet und dort "gesammelt".

Wenn der Fluidpegel in der Luftabscheidekammer 13 gefallen ist, kann die Luft dort entfernt werden. Dazu wird im Bilanziersystem eine positive Bilanz (QD>0) eingestellt, das Entlüftungsventil VL geöffnet und (bevorzugt) die Blutpumpe 7 angehalten.

Dadurch wird das Fluid aus dem Dialysator 11 über die Blutleitung 15 zwischen dem Dialysator 11 und der Luftabscheidekammer 13 in die Luftabscheidekammer 13 geleitet. Es verdrängt die Luft aus der Luftabscheidekammer 13.

Bei Bedarf kann der zweite Füllabschnitt beliebig oft wiederholt werden, so dass vorteilhaft auch größere Mengen an Luft aus dem extrakorporalen Blutkreislauf 100 entfernt werden können.

Wie in Fig. 1 ferner gezeigt, weist der extrakorporale Blutkreislauf 100 einen Sensor 17 zum Messen des arteriellen Drucks vor dem Dialysator 11 ("pre filter pressure"), einen Sensor 19 zum Messen des venösen Drucks stromabwärts der Luftabscheidekammer 13 ("pressure return"), einen ersten Multisensor 21 in der arteriellen Blutleitung 1 und einen zweiten Multisensor 23 in der venösen Blutleitung 3 auf.

Der erste Multisensor 21 kann ein Sensor zum Erfassen nur eines der nachfolgend genannten Parameter oder ein (integrierter) Sensor zur simultanen Erfassung mehrerer Parameter, wie des arteriellen Drucks ("pressure access"), der optischen Dichte (OD) und dergleichen und/oder zur Erfassung der in einem Blutleitungsinneren der arteriellen Blutleitung 1 ggf. befindlichen Luftblasen ("Luftblasendetektor", air bubble detector, ABD) sein.

Der zweite Multisensor 23 kann ein Sensor zum Erfassen nur eines der nachfolgend genannten Parameter oder ein (integrierter) Sensor zur simultanen Erfassung mehrerer Parameter, wie des venösen Drucks, der optischen Dichte (OD) und dergleichen und/oder zur Erfassung der in einem Blutleitungsinneren der venösen Blutleitung 3 ggf. befindlichen Luftblasen ("Luftblasendetektor", air bubble detector, ABD) sein.

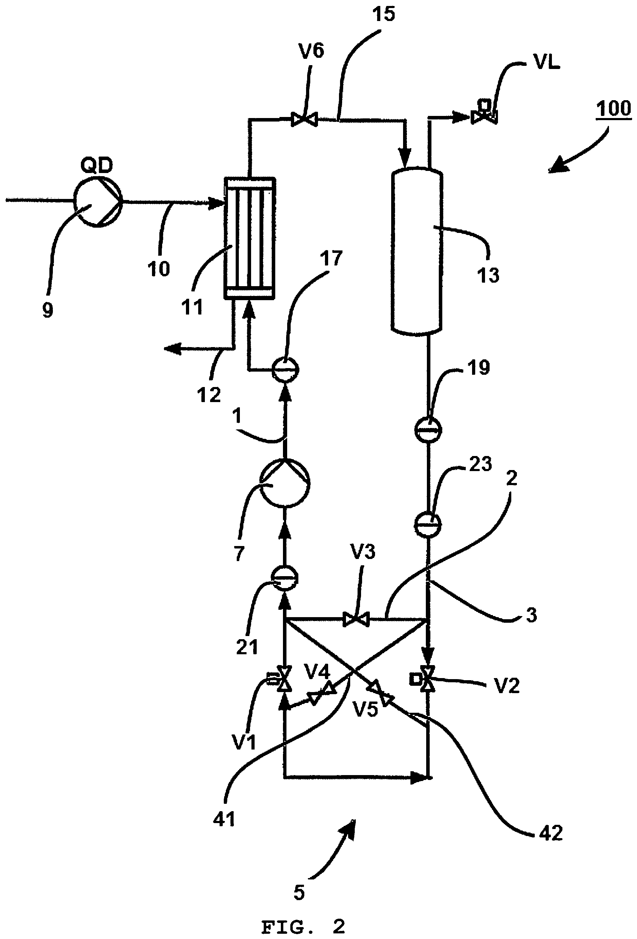

**Fig. 2** zeigt eine zweite erfindungsgemäße Ventilanordnung zur Durchführung eines Füllprozesses bei nicht-okkludierender Blutpumpe in einem ersten Füllabschnitt. Entsprechend ist die Blutpumpe 7 in Fig. 2 als nicht-okkludierende Blutpumpe ausgestaltet, z.B. als Zentrifugalpumpe oder Kreiselpumpe oder Impellerpumpe.

Zur Durchführung auch des anhand von Fig. 2 veranschaulichten Verfahrens sind Patientenkonnektoren (in Fig. 2 nicht gezeigt) mittels des Rezirkulationsadapters 5 kurzgeschlossen. Der Dialysatkreislauf (in Fig. 2 durch die Dialysatzuleitung 10 und die Dialysatableitung 12 angedeutet) ist mit Fluid gefüllt.

Die in Fig. 2 gezeigte Ventilanordnung entspricht im Wesentlichen der Darstellung der Fig. 1. Zusätzlich zu der in Fig. 1 gezeigten Anordnung ist in der Anordnung der Fig. 2 ein weiteres Ventil V6 in der Blutleitung 15 zwischen dem Dialysator 11 und der Luftabscheidekammer 13 angeordnet.

Eine nicht-okkludierende Blutpumpe 7 kann im Stillstand frei von einem Fluid bzw. einer Flüssigkeit durchflossen oder überströmt werden. Eine Flussrichtungsumkehr ist bei einer derartigen Blutpumpe 7 üblicherweise nicht möglich. Der Füllvorgang kann bei Einsatz einer solchen Blutpumpe 7 wie folgt durchgeführt werden:
Analog zur Durchführung in Fig. 1, wird Fluid aus dem Dialysatkreislauf durch Einstellen einer positiven Bilanz in den extrakorporalen Blutkreislauf 100 eingebracht. Im ersten Füllabschnitt ist die Blutpumpe 7 angehalten.

Das Ventil V6 ist zunächst geschlossen, die Ventile V1 und V2 und das Entlüftungsventil VL sind geöffnet. Die Ventile V3, V4 und V5 sind geschlossen. Das hier beschriebene Verfahren kann auch ohne die Ventile V3, V4 und V5 ausgeführt werden.

Das Fluid wird aus dem Dialysatkompartiment über die Membran des Dialysators 11 hindurch auf die Blutseite des Dialysators 11 gepresst und fließt durch die Blutpumpe 7 und die arterielle Blutleitung 1 und die venöse Blutleitung 3 in die Luftabscheidekammer 13.

Sobald in der Luftabscheidekammer 13 ein Fluidpegel erkannt wird, werden die Ventile V1 und V2 geschlossen, das Ventil V6 wird geöffnet. Nun kann das Fluid durch die Blutleitung 15 in die Luftabscheidekammer 13 fließen und die Luft verdrängen. Damit ist der erste Füllabschnitt beendet.

Im zweiten Füllabschnitt (in Fig. 2 nicht gezeigt) wird zunächst die Dialysatpumpe 9 angehalten bzw. das Bilanziersystem (in Fig. 2 nicht gezeigt) auf neutrale Bilanz eingestellt. Das Entlüftungsventil VL wird geschlossen.

Das Fluid wird mit Hilfe der Blutpumpe 7 bei geöffneten Ventilen V1, V2 und V6 im extrakorporalen Blutkreislauf 100 zirkuliert. Dadurch wird die Restluft im extrakorporalen Blutkreislauf 100 in die Luftabscheidekammer 13 geleitet und dort "gesammelt" .

Wenn der Fluidpegel in der Luftabscheidekammer 13 gefallen ist, kann die Luft dort entfernt werden. Dazu wird im Bilanziersystem eine positive Bilanz eingestellt (d.h. es gilt: QD>0). Das Entlüftungsventil VL wird geöffnet. Die Blutpumpe 7 wird bevorzugt angehalten.

Dadurch wird das Fluid aus dem Dialysator 11 über die Blutleitung 15 zwischen dem Dialysator 11 und der Luftabscheidekammer 13 in die Luftabscheidekammer 13 geleitet. Es verdrängt die Luft aus der Luftabscheidekammer 13.

Bei Bedarf kann der zweite Füllabschnitt beliebig oft wiederholt werden, so dass vorteilhaft auch größere Mengen an Luft aus dem extrakorporalen Blutkreislauf 100 entfernt werden können.

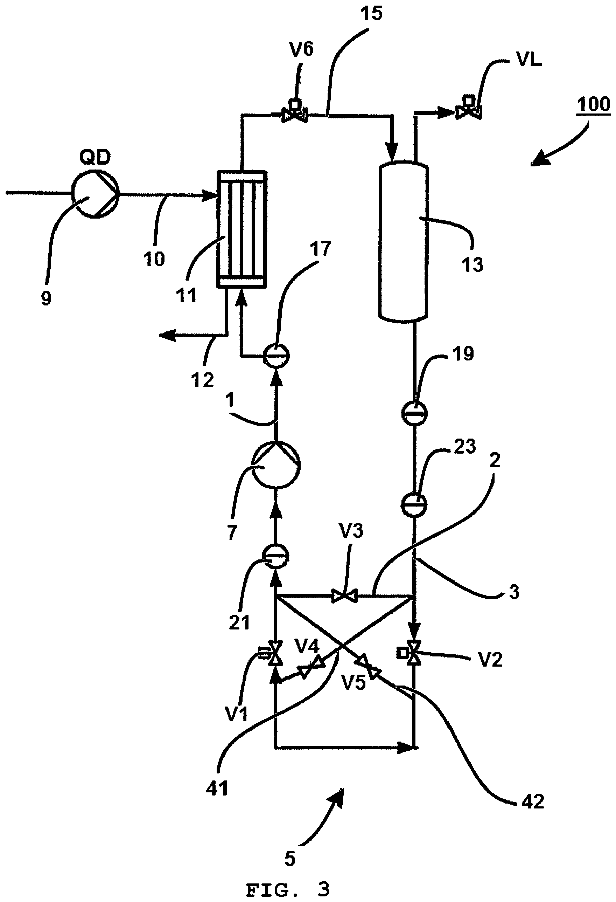

**Fig. 3** zeigt erneut die zweite erfindungsgemäße Ventilanordnung der Fig. 2. Anhand von Fig. 3 wird im Folgenden ein Prozess zum Freispülen von Blutleitungen für den Fall erläutert, dass in wenigstens einer der Blutleitungen ein Flussproblem besteht.

Beim Freispülen ohne Flussprobleme kann es sich um den "normalen" Prozess beim Dekonnektieren eines Patienten handeln.

Die Blutleitungen, d.h. die arterielle Blutleitung 1 und die venöse Blutleitung 3, sind mit dem Gefäßzugang des Patienten, z.B. einem Shunt oder einer Fistel, verbunden. Die Blutpumpe 7 fördert mit einem Fluss QB entgegen der üblichen Flussrichtung (wie sie durch Pfeilrichtung in Fig. 3 angedeutet ist).

Gleichzeitig fördert die Dialysatpumpe 9 Fluid mit einem Fluss QD (mit QD>QB, positive Bilanz) aus dem Dialysatkreislauf (in Fig. 3 durch die Dialysatzuleitung 10 und die Dialysatableitung 12 angedeutet) über die Membran des Dialysators 11 in den extrakorporalen Blutkreislauf 100.

Die Ventile V1, V2 und V6 sind geöffnet. Die Ventile V3, V4 und V5 und das Entlüftungsventil VL sind geschlossen. Damit wird Blut aus dem Dialysator 11 gleichzeitig über die arterielle Blutleitung 1, die venöse Blutleitung 3 und die Blutleitung 15 durch das Fluid verdrängt und dem Patienten zugeführt. Durch die Multisensoren 21 und 23 kann festgestellt werden, wann das Fluid das Blut verdrängt oder ausreichend verdrängt hat.

Eine Steuerung des Blutrückgabeprozesses kann dabei beispielsweise entweder über die Regelung der Flussdifferenz (QD-QB) zwischen dem Fluss QD der Dialysatpumpe 9 und dem Fluss QB der Blutpumpe oder durch Schalten der Ventile V1 und V2 erfolgen.

Bei Verwendung einer nicht-okkludierenden Blutpumpe 7 wird diese beim (Blut-)Rückgabeprozess angehalten und Blut aus den beiden blutführenden Zweigen, d.h. der arteriellen Blutleitung 1 und der venösen Blutleitung 3, inklusive der Blutleitung 15 (zwischen dem Dialysator 11 und der Luftabscheidekammer 13), durch das aus dem Dialysatkompartiment stammende Fluid verdrängt und dem Patienten zurückgegeben. Die Reihenfolge der Rückgabe aus der arteriellen Blutleitung 1 und der venösen Blutleitung 3/Blutleitung 15 kann beliebig wählbar sein. Die Rückgabe wird durch Schalten der Ventile V1 und V2 festgelegt.

In der anhand von Fig. 3 beschrieben Ausführungsform des Verfahrens ist das Ventil V3 in der Regel geschlossen. Das Ventil V3 kann in diesem Verfahren beispielsweise im Falle einer Entkopplung des Patienten bzw. bei dessen Umgehung oder dessen Ausschluss aus der Zirkulation durch den extrakorporalen Blutkreislauf zum Einsatz kommen (d.h. das Ventil V3 ist geöffnet und die Ventile V1, V2, V4 und V5 sind geschlossen), um z.B. Luft abzuscheiden.

In einer weiteren, hier nicht anhand einer weiteren Figur dargestellten Ausführungsform des erfindungsgemäßen Verfahrens wird ein extrakorporaler Blutkreislauf bei Blockade einer der beiden Blutleitungen freigespült, wie im Folgenden beschrieben. Zum besseren Verständnis des Zusammenwirkens der einzelnen Bauteile wird auf die Darstellung des extrakorporalen Blutkreislaufs 100 gemäß der Fig. 1 bis 3 verwiesen.

Dieser Freispülprozess kann zum Einsatz kommen, wenn entweder wenigstens eine der beiden Blutleitungen oder einer der Katheterschenkel blockiert ist. Durch den Freispülprozess wird im Falle einer Blockade vorteilhaft eine zumindest teilweise erfolgende Blutrückgabe ermöglicht, welche bei den sonst üblichen Verfahren nicht oder höchstens eingeschränkt möglich ist.

Für die Durchführung des Freispülens in der hier beschriebenen Ausführung werden die Ventile V3 bis V6 benötigt.

Die Blutleitungen, d.h. die arterielle Blutleitung 1 und die venöse Blutleitung 3, sind jeweils mit dem entsprechenden Gefäßzugang des Patienten verbunden.

Die Blutpumpe 7 fördert mit einem Fluss QB entgegen der üblichen Flussrichtung. Gleichzeitig fördert die Dialysatpumpe 9 mit einem Fluss QD, wobei QD>QB gilt (positive Bilanz), Fluid aus dem Dialysatkreislauf über die Membran des Dialysators 11 in den extrakorporalen Blutkreislauf 100.

Durch den Ventilblock V1-V5, d.h. den Rezirkulationsadapter 5, kann der Gesamtfluss auf eine der beiden Blutleitungen gelegt oder geleitet werden. Ist zum Beispiel der arterielle Schenkel des Katheters verblockt, so kann die Blutrückgabe über die venöse Blutleitung 3 erfolgen: Die Ventile V1, V3 und V4 und das Entlüftungsventil VL sind dabei geschlossen, die Ventile V2 und V5 sind geöffnet. Das Fluid verdrängt das Blut u.a. aus dem Dialysator 11 und der venösen Luftabscheidekammer 13 und wird durch die freie Blutleitung (im gewählten Beispiel die venöse Blutleitung 3) dem Patienten zurückgegeben. Auf diese Weise kann ein Blutverlust bei Verblocken einer Blutleitung vorteilhaft minimiert werden. Durch Schalten der Ventile V5 und V6 bzw. durch geeignete Wahl der Flussdifferenz QD-QB können die beiden Zweige bzw. Blutleitungen - die arterielle Blutleitung 1 und die venöse Blutleitung 3 / Blutleitung 15 - (stromaufwärts und stromabwärts des Dialysators 11) freigespült werden.

In einer weiteren möglichen Ausführung kann alternativ auch bei stehender Blutpumpe 7 (vorzugsweise bei alleinigem Fluss QD der Dialysatpumpe 9) dem Patienten Blut zurückgegeben werden. Im Wesentlichen betrifft dies Blut stromabwärts des Dialysators 11. Diese Ausführung kann sinnvoll sein, wenn der Eingang des Dialysators 11 verblockt ist. Sollte nur der Eingang des Dialysators verblockt sein, können beide Blutleitungen (arterielle Blutleitung 1 und venöse Blutleitung 3 / Blutleitung 15) durch entsprechendes Schalten des Rezirkulationsadapters 5 nacheinander freigespült werden.

In einer weiteren möglichen Ausführung des erfindungsgemäßen Verfahrens kann alternativ mit gleichen Flussraten QD, QB der Pumpen, also bei etwa oder genau QB=QD, dem Patienten Blut zurückgegeben werden. Die Blutpumpe 7 dreht hierbei rückwärts. Nur der Teil stromaufwärts des Dialysators 11 wird freigespült. Dies kann vorteilhaft sein, wenn eine Blockade zwischen dem Ausgang des Dialysators 11 und dem Rezirkulationsadapter 5 vorliegt.

In einer weiteren möglichen Ausführung des erfindungsgemäßen Verfahrens können alternativ bei einer nicht-okkludierenden Blutpumpe 7 die beiden Zweige (arterielle Blutleitung 1 und venöse Blutleitung 3 / Blutleitung 15) (stromauf- und stromabwärts des Dialysators 11) nacheinander freigespült werden. Dazu wird wieder eine positive Bilanz (d.h. es gilt: QD>QB) eingestellt und damit Fluid über die Membran des Dialysators 11 in den extrakorporalen Blutkreislauf 100 gefördert. Durch geeignetes Schalten der Flusswege mittels der Ventile V1 bis V6 können die nicht blockierten Blutleitungen nacheinander freigespült werden (analog zu den drei zuvor beschriebenen Ausführungsformen).

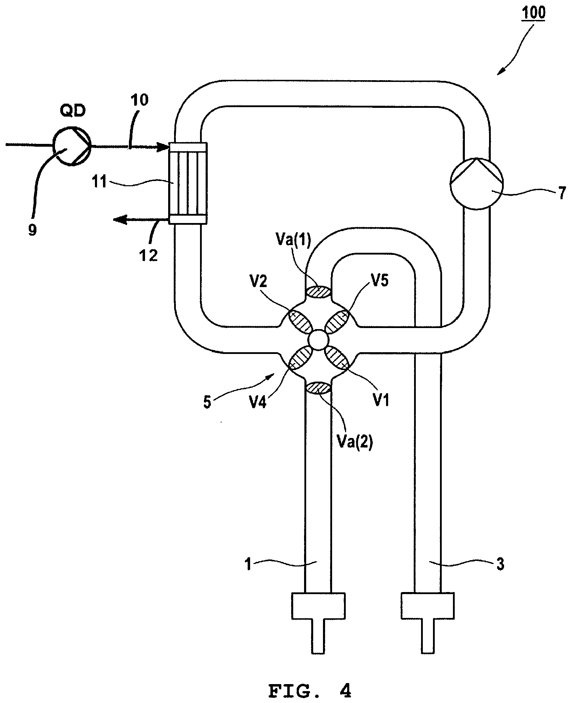

Die Figuren 4 bis 6 zeigen beispielhaft schematische Ausführungsformen eines Rezirkulationsadapters 5, welcher ein Beispiel einer erfindungsgemäßen Ventilanordnung darstellt.

Der Rezirkulationsadapter 5 oder Ventilblock kann Teil einer Blutkassette, z.B. in Form eines Einmalartikels oder einer Disposable-Kassette, sein.

Insbesondere können alle oben genannten Ventile, ganz besonders die Ventile V1 bis V5, Teil eines Einmalartikels oder in einen solchen integriert sein.

In einer weiteren möglichen Ausgestaltungen des Verfahrens kann alternativ mit gleichen Flussraten QD, QB der Pumpen, also bei etwa oder genau QB=QD, dem Patienten Blut zurückgegeben werden. Die Blutpumpe 7 dreht hierbei rückwärts. Nur der Teil stromaufwärts des Dialysators 11 wird freigespült. Dies kann vorteilhaft sein, wenn eine Blockade zwischen dem Ausgang des Dialysators 11 und dem Rezirkulationsadapter 5 vorliegt.

In einer weiteren möglichen Ausgestaltungen des Verfahrens können alternativ bei einer nicht-okkludierenden Blutpumpe 7 die beiden Zweige (arterielle Blutleitung 1 und venöse Blutleitung 3 / Blutleitung 15) (stromauf- und stromabwärts des Dialysators 11) nacheinander freigespült werden. Dazu wird wieder eine positive Bilanz (d.h. es gilt: QD>QB) eingestellt und damit Fluid über die Membran des Dialysators 11 in den extrakorporalen Blutkreislauf 100 gefördert. Durch geeignetes Schalten der Flusswege mittels der Ventile V1 bis V6 können die nicht blockierten Blutleitungen nacheinander freigespült werden (analog zu den drei zuvor beschriebenen Ausführungsformen).

Die Figuren 4 bis 6 zeigen beispielhaft schematische Ausführungsformen eines Rezirkulationsadapters 5, welcher ein Beispiel einer erfindungsgemäßen Ventilanordnung darstellt.

Der Rezirkulationsadapter 5 oder Ventilblock kann Teil einer Blutkassette, z.B. in Form eines Einmalartikels oder einer Disposable-Kassette, sein.

Insbesondere können alle oben genannten Ventile, ganz besonders die Ventile V1 bis V5, Teil eines Einmalartikels oder in einen solchen integriert sein.

Jedoch ist die Erfindung nicht darauf beschränkt, dass alle oder manche Ventile auf oder in einem Rezirkulationsadapter 5 wie im Beispiel der Figuren gezeigt angeordnet sein müssen. Sie können auch ohne eine Einrichtung, die als Adapter ausgestaltet ist, verwirklicht sein.

Bei den in den Fig. 4 bis 6 gezeigten Ausführungsformen sind die nicht-geschalteten Fluidwege vorzugsweise ganz oder im Wesentlichen totraumfrei, so dass vorteilhaft kein Blut oder kein gerinnungsrelevantes Blutvolumen z.B. in einem Ventil- oder Schlauchstück steht oder darin eingeschlossen wird. Der gesamte Ventilraum kann bei strömendem Blut immer freispülbar sein. Dies kann vorteilhaft dazu beitragen, die Gefahr von Gerinnselbildung zu minimieren oder auszuschließen.

Im Beispiel der **Fig. 4** sind die Ventilfunktionen der Ventile V1, V2, V4 und V5 direkt mittels einzelner getrennt voneinander vorliegender Ventile abgebildet (gleiche Bezeichnung der Ventile und der Ventilfunktion). Diese vier Ventile können zum Schalten des Flussweges eingesetzt werden. Die Ventile können in bestimmten erfindungsgemäßen Ausführungsformen jeweils unabhängig voneinander schaltbar sein. Dies kann den Vorteil haben, dass beliebige Flusswege mit dieser Anordnung geschaltet werden können. Die einzelnen Ventile können dabei z.B. Membranventile sein. Die Ventile können wie in der Schrift DE 100 46 651 A1 oder wie in der Schrift DE 102 39 597 A1 beschrieben ausgestaltet sein. Auf die diesbezügliche Offenbarung der vorstehend genannten Dokumente wir hiermit jeweils vollinhaltlich Bezug genommen.

Bei allen oder manchen der Ventile kann es sich um aktive Ventile handeln. In manchen erfindungsgemäßen Ausführungsformen sind die Ventile vorgesehen, maschinell angesteuert zu werden. Sie können eine entsprechende Einrichtung zum Ansteuern aufweisen. In bestimmten Ausführungsformen sind diese Ventile nicht manuell betätigbar oder werden nicht als solche eingesetzt. In manchen erfindungsgemäßen Ausführungsformen sind die Ventile keine Rückschlagventile.

Die Funktion des in Fig. 4 nicht bezeichneten Ventils V3 wird dadurch erreicht, dass entweder V2 und V5 geöffnet und Va(1), V1 und V4 hingegen geschlossen, oder, alternativ, V1 und V4 geöffnet und Va(2), V2 und V5 geschlossen sind. Die Ventile Va(1) und Va(2) können in einem Aufbau gemeinsam vorhanden sein, müssen dies jedoch nicht.

Auch diese Anordnung kann integraler Bestandteil einer Kassette und/oder eines Einwegartikels sein.

Das Ventil V3 kann ein Phantomventil sein. Ein "Phantomventil", wie es hierin verwendet wird, kann wie oben erläutert ein Element mit einer mittels Aktor erreichbaren Aktor-Fläche (beispielsweise eine Aktor-Membran) sein, welches die Funktion eines Ventils übernehmen kann. Beispiele für geeignete Phantomventile können den o.g. Anmeldungen der vorliegenden Anmelderin DE 10 2009 018 664 A1, DE 10 2009 024 468 A1 und der DE 10 2009 012 632 A1 entnommen werden.

Die Ventile Va(1) und Va(2) können von ihrer Bauweise her analog zu Ventilen wie in der Schrift DE 100 46 651 A1 oder DE 102 39 597 A1 beschrieben ausgeführt sein. Auf die diesbezügliche Offenbarung der vorstehend genannten Dokumente wir hiermit jeweils vollinhaltlich Bezug genommen.

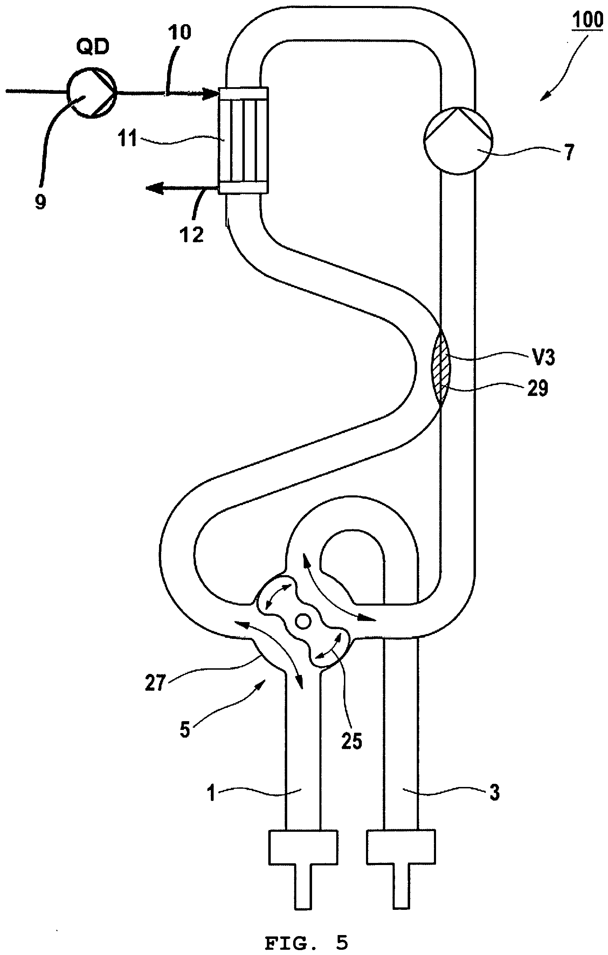

In der in **Fig. 5** gezeigten Ausführung des Rezirkulationsadapters 5 sind die Ventilfunktionen der Ventile V1, V2, V4 und V5 mittels eines Rotationselements oder drehbaren Elements 25 abgebildet. Die Drehbarkeit des Elements 25 ist in Fig. 5 durch die gebogenen Doppelpfeile im Rezirkulationsadapter 5 angedeutet. Durch Verdrehen des Elements 25 um vorgegebene Winkel in einem Gehäuse 27 können die Flusswege entsprechend umgeschaltet bzw. blockiert oder freigegeben werden, ggf. mit Zwischenstufen oder -stellungen zwischen offen und geschlossen. Für die Funktion des Ventils V3 werden im nicht beschränkend zu verstehenden Beispiel der Fig. 5 die Flusswege der Leitungen zum Dialysator 11 und zur Blutpumpe 7 derart zusammengeführt oder verbunden, dass diese nur durch einen, vorzugsweise einfachen, Steg 29 voneinander getrennt sind. Durch ein Element des Ventils V3, welches die Verbindung der beiden Leitungen über den Steg 29 abdichtet oder freigibt, kann entsprechend die Kurzschlussfunktion des Ventils V3 abgebildet werden.

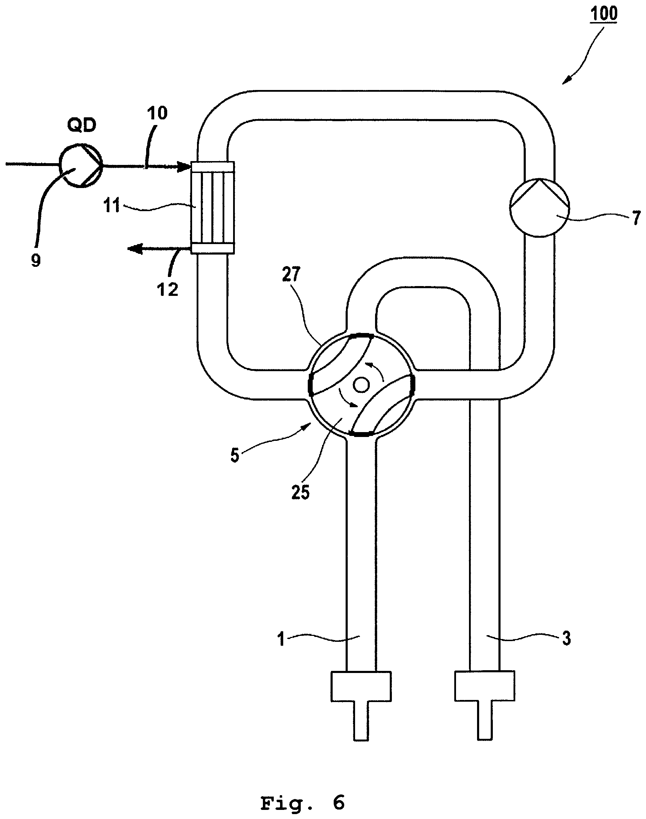

In der in **Fig. 6** gezeigten Ausführung werden mittels des drehbaren Elements 25 die Funktionen der Ventile V1, V2, V4 und V5 ermöglicht. Fig. 6 zeigt eine alternative Ausführungsform des drehbaren Elements 25 der Fig. 5. Die Ventilfunktion des Ventils V3 könnte alternativ analog zur Ausgestaltung des zuvor genannten Ausführungsbeispiels der Fig. 5 ausgeführt werden.

**Bezugszeichenliste**

| **Bezugszeichen** | **Beschreibung** |
|---|---|
| 100 | extrakorporaler Blutkreislauf |
| 1 | arterielle Blutleitung |
| 2 | Blutleitung zwischen der arteriellen |
| | Blutleitung und der venösen Blutleitung |
| 3 | venöse Blutleitung |
| 41 | erste arteriovenöse Blutleitung |
| 42 | zweite arteriovenöse Blutleitung |
| 5 | Rezirkulationsdapter |
| 7 | Blutpumpe |
| 9 | Dialysatpumpe |
| 10 | Dialysatzuleitung |
| 11 | Dialysator |
| 12 | Dialysatableitung |
| 13 | Luftabscheidekammer |
| 15 | Blutleitung zwischen Dialysator und Luftabscheidekammer |
| 17 | Sensor zum Messen des arteriellen Drucks vor dem Dialysator |
| 19 | Sensor zum Messen des venösen Drucks nach der Luftabscheidekammer |
| 21 | erster Multisensor in der arteriellen Leitung |
| 23 | zweiter Multisensor in der venösen Leitung |
| 25 | Drehbares Element |
| 27 | Gehäuse |
| 29 | Steg |
| V1 | Ventile |
| V2 | |
| V3 | |
| V4 | |
| V5 | |
| V6 | |
| Va (1) | |
| Va (2) | |
| VL | Entlüftungsventil |
| QB | Fluss der Blutpumpe |
| QD | Fluss der Dialysatpumpe |

## Patentansprüche

1. Ventilanordnung zur Verwendung in einem extrakorporalen Blutkreislauf (100), welcher wenigstens eine arterielle Blutleitung (1) und wenigstens eine venöse Blutleitung (3) aufweist, wobei die Ventilanordnung wenigstens aufweist:
- ein erstes Ventil (V1), welches in der arteriellen Blutleitung (1) angeordnet ist, ein zweites Ventil (V2), welches in der venösen Blutleitung (3) angeordnet ist, ein viertes Ventil (V4), welches in einer ersten arteriovenösen Verbindungsleitung (41) zwischen arterieller Blutleitung (1) des extrakorporalen Blutkreislaufs (100) und venöser Blutleitung (3) des extrakorporalen Blutkreislaufs (100) angeordnet ist, sowie ein fünftes Ventil (V5), welches in einer zweiten arteriovenösen Verbindungsleitung (42) zwischen der arteriellen Blutleitung (1) und der venösen Blutleitung (3) angeordnet ist; **gekennzeichnet durch**
- ein drittes Ventil (V3), welches zum Herstellen einer Fluidverbindung in einer Blutleitung (2) zwischen arterieller Blutleitung (1) und venöser Blutleitung (3) des extrakorporalen Blutkreislaufs (100) angeordnet ist.

2. Ventilanordnung nach Anspruch 1, welche ein sechstes Ventil (V6) aufweist, welches zwischen einer Blutbehandlungseinrichtung und einer Luftablasseinrichtung des extrakorporalen Blutkreislaufs (100) angeordnet ist.

3. Ventilanordnung nach einem der vorangegangenen Ansprüche, welche zusätzlich ein Entlüftungsventil (VL) zum Herstellen einer Verbindung zwischen einem Inneren des extrakorporalen Blutkreislaufs (100) und einem Äußeren hiervon aufweist.

4. Ventilanordnung nach einem der vorangegangenen Ansprüche, wobei manche oder alle der Ventile der Gruppe, welche das erste, zweite, dritte, vierte, fünfte und sechste Ventil (V1 bis V6) sowie das Entlüftungsventil (VL) umfasst, aktive oder gesteuerte Ventile sind.

5. Ventilanordnung nach einem der vorangegangenen Ansprüche, ausgestaltet und vorgesehen zur Verwendung mit einer nicht-okkludierenden Pumpe zum Fördern des zu behandelnden Fluids.

6. Ventilanordnung nach einem der vorangegangenen Ansprüche, mit einer Einrichtung zum Steuern oder Regeln der Ventilfunktion von allen oder manchen der Ventile der Gruppe, welche das erste, zweite, dritte, vierte, fünfte und sechste Ventil (V1 bis V6) sowie das Entlüftungsventil (VL) umfasst.

7. Ventilanordnung nach einem der vorangegangenen Ansprüche, wobei wenigstens das erste, das zweite, das vierte und das fünfte Ventil (V1, V2, V4, V5) in einem gemeinsamen Trägermaterial vorliegen.

8. Ventilanordnung nach einem der vorangegangenen Ansprüche, wobei wenigstens das dritte Ventil (V3) als Phantomventil vorliegt.

9. Ventilanordnung nach einem der vorangegangenen Ansprüche, welche Sensoren (17, 19, 21, 23) zum Erfassen einer optischen Dichte, eines arteriellen oder venösen Drucks und/oder von Lufteinschlüssen aufweisen.

10. Ventilanordnung nach einem der vorgegangenen Ansprüche, welche eine Einrichtung zum Steuern oder Regeln einer Flussdifferenz (QD-QB) zwischen dem Fluss (QB) der Blutpumpe (7) und dem Fluss (QD) der Dialysatpumpe (9), welche erzeugt wird unter Betreiben einer Blutpumpe (7) und/oder einer Dialysatpumpe (9) und/oder geeigneter Einrichtungen, aufweist.

11. Ventilanordnung nach einem der vorgegangenen Ansprüche, welche in einer Blutkassette ausgestaltet ist oder eine solche aufweist.

12. Ventilanordnung nach einem der vorangegangenen Ansprüche, wobei wenigstens zwei oder mehr Ventile als Abschnitt eines drehbaren Elements (25) mittels Drehung oder Rotation des drehbaren Elements (25) schaltbar ausgestaltet sind.

13. Blutschlauchsatz oder Blutkassette mit wenigstens einer Ventilanordnung gemäß einem der Ansprüche 1 bis 12

14. Behandlungsvorrichtung zum Behandeln medizinischer Fluide, welche wenigstens eine Steuer- oder Regeleinrichtung sowie Aktoren aufweist, die dazu vorgesehen und konfiguriert sind, um wenigstens eine Ventilanordnung gemäß einem der Ansprüche 1 bis 12 zu steuern oder zu regeln.

15. Behandlungsvorrichtung nach Anspruch 14, ausgestaltet als Vorrichtung zur extrakorporalen Blutbehandlung, insbesondere als Vorrichtung zur Hämodialyse, Hämofiltration, Hämodiafiltration, Adsorption, Leberersatztherapie, Apherese, Transfusion und dergleichen.

## Claims

1. A valve arrangement for use in an extracorporeal blood circuit (100) comprising at least one arterial blood line (1) and at least one venous blood line (3), wherein the valve arrangement comprises at least:
a first valve (V1) which is arranged in the arterial blood line (1), a second valve (V2) which is arranged in the venous blood line (3), a fourth valve (V4) which is arranged in a first arteriovenous connection line (41) between the arterial blood line (1) of the extracorporeal blood circuit (100) and the venous blood line (3) of the extracorporeal blood circuit (100), and a fifth valve (V5) which is arranged in a second arteriovenous connection line (42) between the arterial blood line (1) and the venous blood line (3);
**characterized in that**
a third valve (V3) which is arranged for establishing a fluid connection in a blood line (2) between the arterial blood line (1) and the venous blood line (3) of the extracorporeal blood circuit (100).

2. The valve arrangement according to claim 1, comprising a sixth valve (V6) which is arranged between a blood treatment device and an air venting device of the extracorporeal blood circuit (100).

3. The valve arrangement according to anyone of the preceding claims, additionally comprising a ventilation valve (VL) for establishing a connection between an interior of the extracorporeal blood circuit (100) and an exterior thereof.

4. The valve arrangement according to anyone of the preceding claims, wherein some or all of the valves of the group comprising the first, the second, the third, the fourth, the fifth and the sixth valve (V1 to V6) as well as the ventilation valve (VL) are active or controlled valves.

5. The valve arrangement according to anyone of the preceding claims, designed and provided for being used with a non-occluding pump for conveying the fluid to be treated.

6. The valve arrangement according to anyone of the preceding claims, comprising a device for controlling or regulating the valve function of all or some of the valves of the group comprising the first, the second, the third, the fourth, the fifth and the sixth valve (V1 to V6) as well as the ventilation valve (VL).

7. The valve arrangement according to anyone of the preceding claims, wherein at least the first, the second, the fourth and the fifth valve (V1, V2, V4, V5) are present in a common support material.

8. The valve arrangement according to anyone of the preceding claims, wherein at least the third valve (V3) is a phantom valve.

9. The valve arrangement according to anyone of the preceding claims, comprising sensors (17, 19, 21, 23) for detecting an optical density, an arterial or venous pressure and/or air inclusions.

10. The valve arrangement according to anyone of the preceding claims, comprising a device for controlling or regulating a flow difference (QD-QB) between the flow (QB) of the blood pump (7) and the flow (QD) of the dialysate pump (9), wherein the flow difference is generated by means of operating a blood pump (7) and/or a dialysate pump (9) and/or appropriate means.

11. The valve arrangement according to anyone of the preceding claims, which is designed or embodied in a blood cassette or comprises such a blood cassette.

12. The valve arrangement according to anyone of the preceding claims, wherein at least two or more valves are designed or embodied as a segment of a rotational element (25) such as to be switchable by means of rotation of the rotational element (25).

13. A blood tubing set or a blood cassette comprising at least one valve arrangement according to anyone of claims 1 to 12.

14. A treatment apparatus for treating medical fluids comprising at least one controlling or regulating device and actuators that are provided and configured for controlling or regulating at least one valve arrangement according to anyone of claims 1 to 12.

15. The treatment apparatus according to claim 14, designed or embodied as an apparatus for extracorporeal blood treatment, in particular as an apparatus for hemodialysis, hemofiltration, hemodiafiltration, adsorption, liver substitution therapy, apheresis, transfusion and the like.

## Revendications

1. Ensemble de vannes destiné à être utilisé dans une circulation de sang extracorporelle (100) comprenant au moins une conduite de sang artérielle (1) et au moins une conduite de sang veineuse (3), l'ensemble de vannes comprenant au moins :
une première vanne (V1) agencée dans la conduite de sang artérielle (1), une seconde vanne (V2) agencée dans la conduite de sang veineuse (3), une quatrième vanne (V4) agencée dans une première conduite de connexion (41) artério-veineuse entre la conduite de sang artérielle (1) de la circulation de sang extracorporelle (100) et la conduite de sang veineuse (3) de la circulation de sang extracorporelle (100), ainsi qu'une cinquième vanne (V5) agencée dans une seconde conduite de connexion (42) artério-veineuse entre la conduite de sang artérielle (1) et la conduite de sang veineuse (3) ;
**caractérisé en ce que**
une troisième vanne (V3) destinée à réaliser une connexion fluidique entre la conduite de sang artérielle (1) et la conduite de sang veineuse (3) de la circulation de sang extracorporelle (100) est agencée dans une conduite de sang (2).

2. L'ensemble de vannes selon la première revendication comprenant une sixième vanne (V6) agencée entre un dispositif de traitement du sang et un moyen d'évacuation de l'air de la circulation de sang extracorporelle (100).

3. L'ensemble de vannes selon l'une quelconque des revendications précédentes comprenant de plus une vanne de purge d'air (VL) destinée à réaliser une connexion entre un intérieur de la circulation de sang extracorporelle (100) et un extérieur de celle-ci.

4. L'ensemble de vannes selon l'une quelconque des revendications précédentes, où certaines ou toutes les vannes du groupe comprenant la première, la seconde, la troisième, la quatrième, la cinquième et la sixième vanne (V1 à V6) ainsi que la vanne de purge d'air (VL) sont des vannes actives ou contrôlées.

5. L'ensemble de vannes selon l'une quelconque des revendications précédentes configuré et prévu pour être utilisé avec une pompe non-occluante pour transporter le fluide à traiter.

6. L'ensemble de vannes selon l'une quelconque des revendications précédentes avec un moyen destiné à contrôler ou régler la fonction de vanne de certaines ou de toutes les vannes du groupe comprenant la première, la seconde, la troisième, la quatrième, la cinquième et la sixième vanne (V1 à V6) ainsi que la vanne de purge d'air (VL).

7. L'ensemble de vannes selon l'une quelconque des revendications précédentes où au moins la première, la seconde, la quatrième et la cinquième vanne (V1, V2, V4, V5) sont présentes dans un matériau support commun.

8. L'ensemble de vannes selon l'une quelconque des revendications précédentes où au moins la troisième vanne (V3) est présente sous forme de vanne fantôme.

9. L'ensemble de vannes selon l'une quelconque des revendications précédentes comprenant des capteurs (17, 19, 21, 23) destinés à détecter une densité optique, une pression artérielle ou veineuse et/ou des inclusions d'air.

10. L'ensemble de vannes selon l'une quelconque des revendications précédentes comprenant un moyen destiné à contrôler ou à régler une différence de flux (QD-QB) entre le flux (QB) de la pompe à sang (7) et le flux (QD) de la pompe à dialysat (9) qui est générée lors du fonctionnement d'une pompe à sang (7) et/ou d'une pompe à dialysat (9) et/ou de moyens adéquats.

11. L'ensemble de vannes selon l'une quelconque des revendications précédentes configurée en tant que ou comprenant une cassette à sang.

12. L'ensemble de vannes selon l'une quelconque des revendications précédentes où au moins deux vannes, ou plus, sont configurées en tant que portion d'un élément rotatif (25) de façon à être commutables au moyen de la rotation de l'élément rotatif (25).

13. Ensemble de tubes à sang ou cassette à sang avec au moins un ensemble de vannes selon l'une quelconque des revendications 1 à 12.

14. Dispositif de traitement destiné à traiter des fluides médicaux comprenant au moins un moyen de contrôle ou de réglage ainsi que des actionneurs prévus et configurés pour contrôler ou régler au moins un ensemble de vannes selon l'une quelconque des revendications 1 à 12.

15. Dispositif de traitement selon la revendication 14 configuré comme dispositif destiné à traiter le sang de façon extracorporelle, en particulier comme dispositif destiné à l'hémodialyse, l'hémofiltration, l'hémodiafiltration, l'adsorption, la thérapie de remplacement du foie, l'aphérèse, la transfusion et similaire.
